# EUROPEAN PATENT APPLICATION

(11) **EP 4 520 208 A1**
(43) Date of publication of application: **12.03.2025**
(21) Application number: 24196888.2
(22) Date of filing: 28.08.2024
(51) Int. Cl.: A41B 13/08, A41D 15/00, A41B 9/08, A61F 13/15, A41B 9/12

(54) **A DRESS WITH A CLOTH EXTENDER**

(30) Priority: 04.09.2023 PH 12023050489
(71) Applicant: Balani, Vashi T., 1209 Metro Manila (PH)
(72) Inventor: Balani, Vashi T., 1209 Metro Manila (PH)
(74) Representative: Weisse, Moltmann & Willems PartGmbB

(57) **Abstract**

A dress with a cloth extender (10), comprising a dress (12), a plurality first set female fastening means (14) attached to the lower front side of the dress, a plurality of first set of male fastening means (16) attached on the lower rear side of the dress, a cloth extender (20) being attachable to the dress and wherein said cloth extender further comprises: a plurality of second-set male fastening means (22) being fixedly attached to a front cloth lining (24), a cloth (26) fixedly sewn in between the front cloth lining and a rear cloth lining (28), a piping cloth (30) sewn on the side of the cloth, a plurality of second-set female fastening means (32) attached to the rear cloth lining, wherein said cloth extender when attached to the dress through the plurality of second-set female fastening means being snapped and attached to the plurality of first-set male fastening means and the plurality of first set female fastening means to the plurality of second-set male fastening means provides an extension and adjustment means to the dress enabling said dress to be usable again.

## Description

### Technical Field

The present invention generally relates to a wearable dress with an attachment. More particularly, a dress with a cloth extender configured as an extension or adjustment to a dress that will make the old or outgrown cloth dress to be usable again.

### Background

A variety of cloth or dresses exists in the prior art, whose primary objective is to provide a means for protecting the human body. From baby up to the stage of adulthood, clothing size is typically based on age. Also, personal fashion is a social-cultural consumerist practice that encodes in people's fashion the representation of many social features and depicts a system characterized by differences in social class, richness, gender, or ethnicity.

Clothes or dresses are sometimes sewn with a snap at the crotch. These snaps help fit the fabric clothing perfectly to the body and help facilitate changing underwear or diapers as it opens and closes in the crotch area. However, given that humans grow bigger and taller rapidly, cloth extenders are beneficial for using dresses for long periods. By making the dress last longer and reusable, helps to reduce carbon footprint. Different companies have different ways of stitching or attaching the snaps; for example, the use of different diameters of the snaps used in the dress and the extender can cause a mismatch. Also, using different quality snaps, as snaps made of plastic; some are nickel plated, and some are metal plated, etc., contributes to the action of mismatching the extenders with the dress. Even a slight change in the position of the attachment of the snaps, either in the dress or the extender, would cause the failure of the attachment of the extender to the dress. Thus, snaps mismatches when bought from various businesses.

### Object of the invention

The object of the invention was therefore to eliminate the disadvantages of the prior art and in particular to provide clothes or dresses with cloth extenders with a standard arrangement of attachment means.

### Disclosure of the invention

The object of the invention is solved by the features of the independent claims. Advantageous embodiments of the invention are described in the dependent claims.

The present invention generally relates to a wearable dress with an attachment. More particularly, a dress with a cloth extender configured as an extension or adjustment to a dress that will make the old or outgrown cloth dress to be usable again.

Furthermore, the present invention generally relates to a wearable dress with an attachment, more particularly to be known as a dress with a cloth extender. It comprises a dress, a plurality first set female fastening means attached to the lower front side of the dress, a plurality of first set of male fastening means attached on the lower rear side of the dress, and a cloth extender which is attachable to the dress. The cloth extender comprises a plurality of second-set male fasting means being fixedly attached to a front cloth lining, a cloth fixedly sewn in between the front cloth lining and a rear cloth lining, a piping cloth sewn on the side of the cloth, and a plurality of second-set female fasting means attached to the rear cloth lining.

When the cloth extender is attached to the dress through the plurality of second-set female fasting means being snapped and attached to the plurality of first-set male fastening means and the plurality of first set female fasting means to the plurality of second-set male fastening means, the cloth extender provides extension and adjustment means to the dress enabling the dress to be usable again.

Since that human grows fast, the garment soon comes to the stage of being discarded. However, using dresses or clothes with cloth extenders according to the invention will bring new life to the garment. With the preferred cloth extender, the end user will be more comfortable, and with the extender attached to the clothing, the muscles will lose due to the release of pressure from the outgrown clothing, making the user more comfortable wearing the clothing or dress.

In this respect, particularly the provision of a cloth extender with corresponding male and female fastening means allows for a versatile adjustment of the dress size, accommodating changes in the wearer's body size or preference for dress fit. The attachment of the cloth extender to the dress through fastening means offers a simple and user-friendly method to modify the dress length or width without the need for permanent alterations or sewing skills.

According to the invention, the dress can be designed as a romper for children or babies or, for example, as underwear for adults. However, the design is not limited to these options and may apply to any suitable garment. Moreover, the dress is preferably intended for babies, toddlers, children or adults.

The dress may have (first set) fastening means. These fastening means comprise a female and/or a male component, the interaction between the female and male components providing a secure but releasable connection. Such fastening means may be, for example, snaps, hooks, zips, Velcro or other similar. A combination of different fastening means may also be provided.

Preferably, the fastening means are attached to the lower front or lower rear side of the dress. For example, they may be sewn into the dress. "Lower front" and "lower rear" refer to specific regions of the dress in relation to the wearer's body. The term "lower front" describes the area of the dress located at the front of the body, near the lower portion or hemline, typically around the waist, hips, or thighs, depending on the dress length. Similarly, "lower rear" refers to the back side of the dress, near the lower hemline, which would correspond to the area around the wearer's lower back, hips, or buttocks. The fastening means can, for example, be positioned or spaced at a distance of 1 mm to 50 mm from the hemline of the dress.

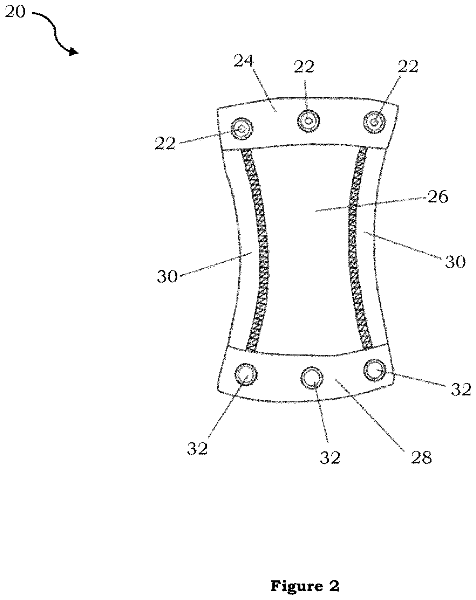

The cloth extender is, preferably a piece of fabric or cloth that essentially may have an elongated, rectangular shape. In a top view, the cloth extender is particularly hourglass-shaped or waisted (see Fig. 2). It is narrower in the middle and wider at the ends and edges. This shape allows for a better fit between the legs and provides increased comfort and freedom of movement.

The cloth extender includes preferably second-set male and female fastening components, which can be coupled to the corresponding fastening means of the dress. These components may include, for example, snaps, hooks, zippers, Velcro, or other similar fastening components.

Further, the cloth extender may come in various sizes, allowing for different extenders to be used ,for example, depending on a growth phase of a baby or child, or whether a diaper is being used. For example, the dress can initially be worn without an extender. After a few months, when the child has outgrown the dress, an extender of the first size can be used. As the child continues to grow, a second-size extender can be applied. Additionally, if a baby or child is wearing diapers, an extender can be added to accommodate the extra space needed. Once the child no longer wears diapers, the dress can be worn without the extender, as there is no longer a need for the additional space for the diaper.

The cloth extender may comprise different parts, particularly three sections. The cloth extender can feature cloth linings (first and second section) at the front and rear edges, where the front edge refers to the side that faces the wearer's stomach or abdomen, and the rear edge refers to the side that faces the wearer's back. Between these cloth linings, a piece of fabric or cloth can be sewn (third section). The cloth linings may be made more durable by using different types of fabric or multiple layers compared to the piece of cloth arranged in between the cloth linings.

Additionally, the cloth extender may comprise a piping cloth, which is sewn along the sides of the extender. Piping cloth refers to a narrow strip of fabric that is sewn into the seams to reinforce the edges and provide a more finished, polished appearance. This piping serves several purposes: it enhances the durability of the extender by preventing fraying at the edges, reinforces the structural integrity of the extender, and adds a decorative touch by outlining the shape of the garment. By adding piping, the extender maintains its shape better, especially after repeated use and washing, as the piping helps to prevent the fabric from stretching or distorting. Furthermore, the piping adds comfort by ensuring the edges of the extender remain smooth and don't irritate the wearer's skin.

Particularly, the piping cloth can be provided to cover the side cutting edges of the cloth extender and preventing the cloth from being stretched out when attached to the dress.

Preferably, the cloths used in the invention are stitched using either knit or woven fabrics, and the fabric may be cotton or any kind. The extenders are preferably made from the same fabrics and can be matched in color and the snaps' quality.

In a preferred embodiment the plurality of first set of female fastening means, the plurality of second set of female fastening means, the plurality of first set of male fastening means, and the plurality of second set of male fastening comprise snap buttons. Utilizing snap buttons as the fastening means for the cloth extender ensures a secure and durable connection that can withstand repeated use and the stress of garment movement. The snap buttons provide a quick and easy method for attaching and detaching the cloth extender, making the modification process convenient for the user. Snap buttons are less likely to cause discomfort or irritation to the wearer, as they have a flat profile and do not protrude significantly from the fabric surface.

In a preferred embodiment, the dress and the cloth of the cloth extender have the same kind of color combination. A consistent color combination between the dress and the cloth extender creates a seamless visual extension of the garment, making the addition of the extender less noticeable and more aesthetically pleasing.

In a further preferred embodiment, the dress with a cloth extender comprises six snap buttons, wherein the first set of female fastening means and second set of female fastening means each comprise three female parts of the snap buttons and the first set of male fastening means, and the second set of male fastening each comprise three male parts of the snap buttons. This number of snap buttons has proven to be optimal for several reasons: it ensures a secure and stable attachment between the dress and the extender which prevents accidental detachment during wear. The three snap buttons on each side evenly distribute pressure across the fabric, reducing stress and wear on the garment. Furthermore, the six-snap configuration offers flexibility for incremental adjustments to accommodate different body sizes while enhancing the overall durability and longevity of both the dress and the extender.

The first set of female fastening means, the second set of female fastening means, the first set of male fastening means, and the second set of male fastening means are each arranged such that a virtual connecting line between the respective male or female fastening means runs parallel to an edge of the dress or the cloth extender. Accordingly, all fastening means or snap buttons are located at an equal distance from the edge or hem of the dress or cloth extender. It allows for an even distribution of tension across the fabric, reducing the risk of pulling, warping, or material damage over time.

A virtual connecting line between the respective male or female fastening means is to be understood as follows: Each male or female fastening means, or each male or female part of the snap buttons, has a center point. The line connecting these center points is referred to as the virtual connecting line between the respective male or female fastening means. In other words, each button has a center, and when all of these centers are mentally connected, the result is the virtual connecting line between the respective male or female fastening means. Only the female or male fastening means within a single set are mentally connected to form the virtual line.

The phrase "parallel to an edge of the dress or the cloth extender" refers to the arrangement of the fastening means, such as snap buttons, in a manner where the virtual connecting line formed by linking the centers of the fastening means runs consistently in the same direction as the edge of the dress or the cloth extender. This virtual line remains equidistant from the edge at all points, ensuring a smooth, aligned fit. The edge could refer to the hemline, side seams, or any defined boundary of the garment. By arranging the fastening means parallel to this edge, the fasteners follow the natural contour of the garment, which ensures a consistent attachment while evenly distributing tension across the fabric. This helps maintain the garment's structural integrity and aesthetic appearance.

In a further preferred embodiment, the cloth extender comprises an elastic band in an edge area. The edge area can be defined as the region extending, for example, 5 mm to 20 mm inward from the outermost perimeter or hem of the cloth extender. By incorporating an elastic band in this region, the extender gains flexibility, allowing it to stretch and contract to fit more securely and comfortably. The elastic band helps ensure a snug fit against the wearer's body, preventing the extender from shifting or bunching during movement, and allowing the garment to adapt more easily to changes in body size or shape.

In a further preferred embodiment the cloth extender comprises an absorbent pad, which is either integrated into the cloth or detachably attached to it. The absorbent pad preferably refers to a layer of material designed to absorb moisture, such as sweat or bodily fluids, to keep the wearer dry and comfortable. This pad may be made from materials such as cotton, microfiber, or other absorbent fabrics. When the pad is integrated into the cloth, it is permanently sewn into the cloth or the fabric cloth extender, creating a single, inseparable unit. In the case of a detachable pad, it can be removed through fasteners like Velcro, snaps, or hooks, allowing for flexibility in use.

In a further aspect, the invention relates to a method for adjusting a dress to accommodate a change in the size of a human body, such as that of a baby in the growth phase. This method comprises the following steps. First, a dress with a cloth extender according to the embodiments above is provided. Further, the dress with a cloth extender is used in a first configuration, wherein in the first configuration the cloth extender is detached from the dress. Subsequently, it is determined that the dress no longer fits to the size of the human body in the first configuration. Accordingly, the dress with a cloth extender is used in a second configuration. In the second configuration the cloth extender is attached to the dress to adjust the fit.

The skilled person will recognize that the advantages, technical effects and preferred embodiments discussed in connection with the dress with a cloth extender apply analogously to the method for adjusting a dress to accommodate a change in the size of a human body, such as that of a baby in the growth phase. Likewise, all the advantages, technical effects and preferred embodiments described in connection with the method are transferable to the dress with a cloth extender.

In a further preferred embodiment, the method comprises the steps of determining that the dress with a cloth extender no longer fits in the second configuration, of providing an additional cloth extender that is larger than the initial cloth extender, and of using the dress with the cloth extender in a third configuration, wherein the initial cloth extender is substituted through the additional cloth extender to adjust the fit.

Further examples of embodiments are explained in more detail below with reference to the accompanying drawings. The invention is not intended to be limited solely to these listed examples of embodiments. They merely serve to explain the invention in more detail. The present invention is intended to relate to all objects which the person skilled in the art would use now and, in the future, as obvious to realize the invention.

### Brief Description of the Drawings

**Figure 1** is a front view of a dress and a cloth extender.
**Figure 2** is a perspective view of the cloth extender.

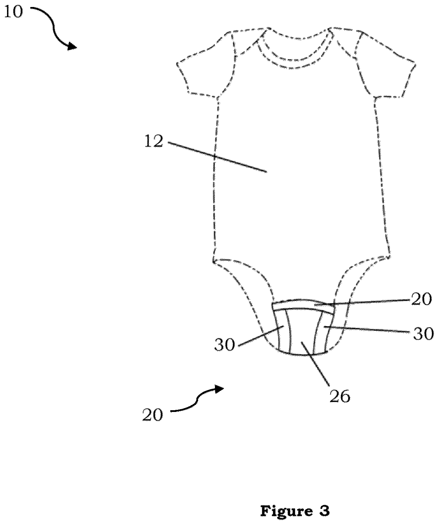

**Figure 3** is a view of the present embodiment of a dress with a cloth extender.

### Detailed Description

Before describing the present invention in more detail, it is to be understood that the phraseologies and terminologies used herein are for the purposes of description and should not be regarded as limiting.

It will be understood that although the terms first, second, third, etc., may be used to describe various elements, these elements should not be limited by these terms. These terms may be used to distinguish one element from another. For example, a first element may be termed a first element without departing from the scope of the present application. As used herein, the term "and/ or" may include all combinations of one or more of the listed items.

Referring now to the drawings, wherein like reference numerals, designate the components or elements throughout the ensuing enabling description, the present invention provides a dress with a cloth extender designated as 10.

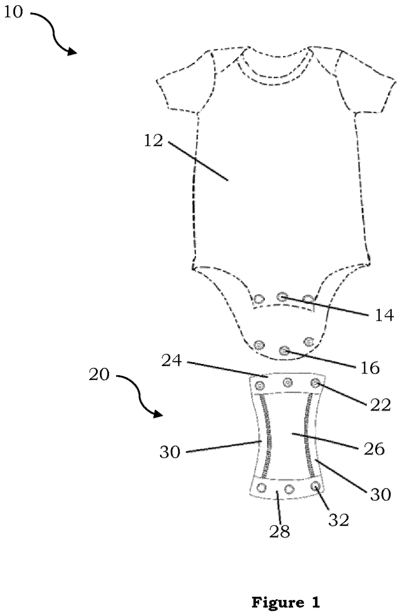

In reference to Figure 1, the dress with a cloth extender 10 comprises a dress 12, a plurality of first set female fastening means 14 attached on the lower front side of the dress 12, a plurality of first set male fastening means 16 attached on the lower rear side of the dress 12, and a cloth extender 20.

The cloth extender 20, as shown in Figure 2, further comprises a plurality of second-set male fasting means 22 being fixedly attached to a front cloth lining 24, a cloth 26 fixedly sewn in between the front cloth lining 24 and a rear cloth lining 28, a piping cloth 30 sewn on the side of the cloth 26 and wherein said piping cloth 30 is provided to cover the side cutting edges of the cloth extender 20 and preventing the cloth 26 to be stretched out when attached to the dress 12, and a plurality of second set female fasting means 32 attached to the rear cloth lining 28. The cloth 12 is preferably cotton or another kind of fabric used for clothes being breathable and absorbent fabric.

When used, the plurality of second-set female fasting means 32 are snapped and attached to the plurality of first-set male fastening means 16, while the plurality of first-set female fasting means 14 are snapped and attached to the plurality of second-set male fastening means 22. The dress with a cloth extender 10 has the same kind of color combination and is made mostly from the same fabric and matched color. Furthermore, said dress with a cloth extender 10 is characterized by said cloth extender 20 being configured as an extension or adjustment to the dress 12 that will make the old or outgrown cloth dress to be usable again.

Lastly, the plurality of first set of female fastening means 14, the plurality of second set of female fastening means 32, the plurality of first set of male fastening means 16, and the plurality of second set of male fastening 22 used snap buttons for the great quality, durability, and strength for rugged use.

Additional advantages and modifications of the present invention will readily occur to those skilled in the art in view of these teachings. The present invention, in its broader aspects, is not limited to the specific details, representative contrivances, and illustrative examples shown and described herein. Accordingly, various modifications may be made without departing from the spirit and scope of the general concept as defined in the appended claims and its equivalents.

### REFERENCE NUMERAL LIST

- 10: dress with a cloth extender
- 12: dress
- 14: first set female fastening means
- 16: first set of male fastening means
- 20: cloth extender
- 22: second-set male fasting means
- 24: front cloth lining
- 26: cloth
- 28: rear cloth lining
- 30: piping cloth
- 32: second-set female fasting means

## Claims

**1.** A dress with a cloth extender (10), comprising;
a dress (12),
a plurality first set female fastening means (14) attached to the lower front side of the dress (12),
a plurality of first set of male fastening means (16) attached on the lower rear side of the dress (12),
a cloth extender (20) being attachable to the dress (12) and wherein said cloth extender (20) further comprises:
a plurality of second-set male fasting means (22) being fixedly attached to a front cloth lining (24),
a cloth (26) fixedly sewn in between the front cloth lining (24) and a rear cloth lining (28),
a piping cloth (30) sewn on the side of the cloth (26),
a plurality of second-set female fasting means (32) attached to the rear cloth lining (28),
wherein said cloth extender (20) when attached to the dress (12) through the plurality of second-set female fasting means (32) being snapped and attached to the plurality of first-set male fastening means (16) and the plurality of first set female fasting means (14) to the plurality of second-set male fastening means (22) provides an extension and adjustment means to the dress (12) enabling said dress 12 to be usable again.

**2.** The dress with a cloth extender (10) according to claim 1, wherein said plurality of first set of female fastening means (14), the plurality of second set of female fastening means (32), the plurality of first set of male fastening means (16), and the plurality of second set of male fastening (22) are snap buttons.

**3.** The dress with a cloth extender (10) according to claim 1 or claim 2, wherein said dress (12) and cloth (26) have the same kind of fabric.

**4.** The dress with a cloth extender (10) according to any of the preceding claims, wherein said dress (12) and said cloth (26) have the same kind of color combination.

**5.** The dress with a cloth extender (10) according to any of the preceding claims, wherein said piping cloth (30) is provided to cover the side cutting edges of the cloth extender (20) and preventing the cloth (26) from being stretched out when attached to the dress (12).

**6.** The dress with a cloth extender (10) according to claim 2, wherein the dress with a cloth extender (10) comprises six snap buttons, wherein the first set of female fastening means (14) and second set of female fastening means (32) each comprise three female parts of the snap buttons and the first set of male fastening means (16), and the second set of male fastening (22) each comprise three male parts of the snap buttons.

**7.** The dress with a cloth extender (10) according to claim 6, wherein the first set of female fastening means (14), the second set of female fastening means (32), the first set of male fastening means (16), and the second set of male fastening (22) are each arranged such that a virtual connecting line between the respective male or female fastening means (14, 16, 22, 32) runs parallel to an edge of the dress (12) or the cloth extender (20).

**8.** The dress with a cloth extender (10) according to any of the preceding claims, wherein the cloth extender (20) comprises an elastic band in an edge area.

**9.** The dress with a cloth extender (10) according to any of the preceding claims, wherein the cloth extender (20) comprises an absorbent pad, which is either integrated into the cloth (26) or detachably attached to it.

**10.** The dress with a cloth extender (10) according to any of the preceding claims, wherein the dress (12) is intended for babies, toddlers or children.

**11.** The dress with a cloth extender (10) according to any of the preceding claims 1 - 9, wherein the dress (12) is intended for adults.

**13.** A method for adjusting a dress to accommodate a change in the size of a human body, such as that of a baby in the growth phase, comprising
a) providing a dress with a cloth extender (10) according to any of the preceding claims;
b) using the dress with a cloth extender (10) in a first configuration, wherein the cloth extender (20) is detached from the dress (12);
c) determining that the dress no longer fits in the first configuration;
d) using the dress with a cloth extender (10) in a second configuration, wherein the cloth extender (20) is attached to the dress (12) to adjust the fit.

**14.** The method according to claim 13, wherein the method further comprises the following steps:
e) determining that the dress with a cloth extender (10) no longer fits in the second configuration;
f) providing an additional cloth extender (20) that is larger than the initial cloth extender (20);
g) using the dress with the cloth extender (10) in a third configuration, wherein the initial cloth extender (20) is substituted through the additional cloth extender (20) to adjust the fit.
